# EUROPEAN PATENT APPLICATION

(11) **EP 4 589 000 A1**
(43) Date of publication of application: **23.07.2025**
(21) Application number: 25152907.9
(22) Date of filing: 20.01.2025
(51) Int. Cl.: C12M 3/06, C12M 1/00, C12M 1/12, C12M 1/42

(54) **MICROFLUIDIC CHIP AND METHOD FOR PRODUCING SPHEROID OR ORGANOID IN VITRO MODEL USING THE SAME**

(30) Priority: 18.01.2024 KR 20240007773; 25.07.2024 KR 20240098734
(71) Applicant: Korea University Research and Business Foundation, Seoul 02841 (KR)
(72) Inventor: CHUNG, Seok, 04585 Seoul (KR); KANG, Seok-Hyeon, 02858 Seoul (KR); KIM, Jin-A, 02714 Seoul (KR); AHN, Jin-Chul, 05666 Seoul (KR); BAE, Hyun-Jun, 03934 Seoul (KR); LEE, Dain, 02754 Seoul (KR)
(74) Representative: Dehns

(57) **Abstract**

The present invention relates to a microfluidic chip, which includes cell inlets (11) into which a fluid containing cells is injected, and a cell channel (12) connected thereto; and extra cellular matrix (ECM) inlets (21) into which a fluid containing an ECM component is injected, and an ECM channel (22) connected thereto, wherein the cell channel (12) is located adjacent to a part of one side of the ECM channel (22), and a micro barrier (40) with a lower height than these channels (12, 22) is located between them and formed by arranging a plurality of concave patterns (41), curved in the direction of the ECM channel (22), at regular intervals. The present invention also relates to a method of forming a spheroid or organoid *in vitro* model using the same.

## Description

### BACKGROUND

### 1. Field of the Invention

The present invention relates to a microfluidic chip using a micro barrier with concave patterns and a method of producing a spheroid or organoid *in vitro* model using the same.

### 2. Discussion of Related Art

To treat human diseases, numerous drugs are being developed and evaluated, and research on *in vitro* human tissue and organ models is being conducted to develop drugs. Among the models, there are spheroids and organoids, which are three-dimensionally formed by gathering cells and can provide a more complex and diverse microenvironment than two-dimensionally cultured cells. However, general spheroids and organoids have the problem that the absence of perfusable three-dimensional blood vessels makes it difficult to smoothly supply oxygen and nutrients and to create an internal environment of a model.

To overcome these limitations, models for co-culturing spheroids, organoids and a vascular network on a microfluidic chip have been developed. However, conventional co-culture models have a common disadvantage that the process of injecting spheroids and organoids into a chip is considerably complex and difficult. In addition, there was also difficulty in controlling the numbers and locations of spheroids and organoids when the spheroids and organoids were cultured in conventional microfluidic chips.

Korean Patent No. 10-1949612 (2019.02.12.) discloses a prior art arrangement.

### SUMMARY OF THE INVENTION

The present invention is directed to providing a spheroid or organoid microfluidic chip, which can improve analysis convenience and form various co-culture models. For this, the spheroid or organoid microfluidic chip includes cell inlets into which a fluid containing cells is injected, and a cell channel connected thereto; and extra cellular matrix (ECM) inlets into which a fluid containing an ECM component is injected, and an ECM channel connected thereto. Here, the cell channel is located adjacent to a part of one side of the ECM channel, and a micro barrier with a lower height than these channels is located between them. The micro barrier is formed by arranging a plurality of concave patterns, curved in the direction of the ECM channel, at regular intervals.

However, technical problems to be solved in the present invention are not limited to the above-described problems, and other problems which are not described herein will be fully understood by those of ordinary skill in the art from the following descriptions.

According to an aspect of the present invention, there is provided a spheroid or organoid microfluidic chip, which includes cell inlets into which a fluid containing cells is injected, and a cell channel connected thereto; and extra cellular matrix (ECM) inlets into which a fluid containing an ECM component is injected, and an ECM channel connected thereto. Here, the cell channel is located adjacent to a part of one side of the ECM channel, and a micro barrier with a lower height than these channels is located between them and formed by arranging a plurality of concave patterns, curved in the direction of the ECM channel, at regular intervals.

Between the cell channel and the ECM channel, where the micro barrier is not placed, the cells and the ECM component may interact with each other.

The height of the micro barrier may be 1/5 to 1/2 of the height of these channels.

In the micro barrier, both ends of a concave pattern may be connected to different chamfers.

In the micro barrier, the diameter of a concave pattern may be 1/5 to 1/3 of the maximum width of the ECM channel.

In the micro barrier, the gap between concave patterns may range from 100 µm to 5,000 µm.

The cells may be cancer cells or neuronal cells.

A second cell channel, located adjacent to a part of the other side of the ECM channel, may be further included.

Between the second cell channel and the ECM channel, a second micro barrier with a lower height than these channels may be located and formed by arranging a plurality of concave patterns, curved in the direction of the ECM channel, at regular intervals.

Second cells are the same or different type of the above-mentioned cells, and may be one or more types of co-culturing cells selected from the group consisting of cancer cells, neuronal cells, and vascular endothelial cells.

According to another aspect of the present invention, there is provided a method of producing a spheroid or organoid *in vitro* model, which includes (a) injecting a fluid containing cells into a cell inlet of a microfluidic chip; and (b) forming or culturing a spheroid or organoid with the injected cells at the locations of concave patterns that are arranged in the cell channel at regular intervals.

### BRIEF DESCRIPTION OF THE DRAWINGS

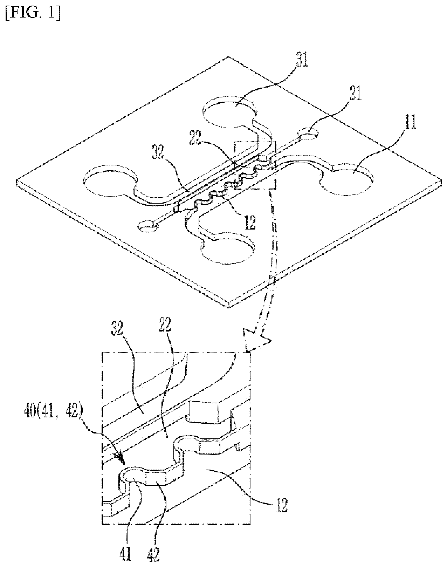

FIG. 1 schematically shows a microfluidic chip according to one embodiment of the present invention and a method of producing a spheroid or organoid *in vitro* model using the same.

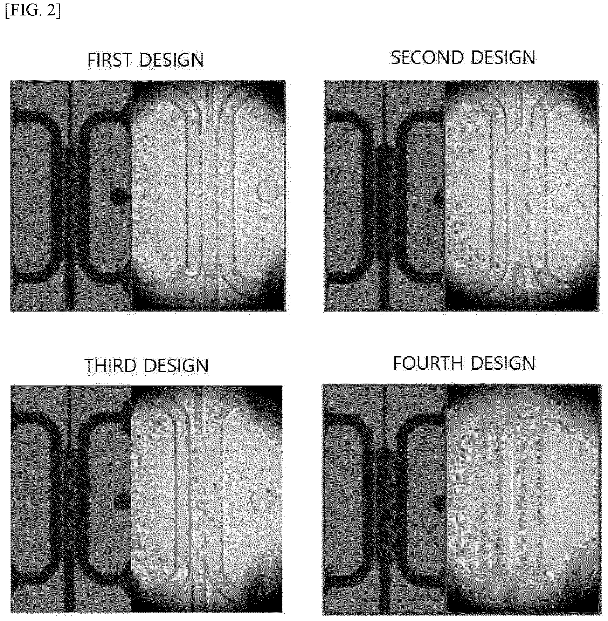

FIG. 2 shows design changes for optimizing the diameter (D) of a concave pattern and the maximum width (W) of an ECM channel in the microfluidic chip according to one embodiment of the present invention.

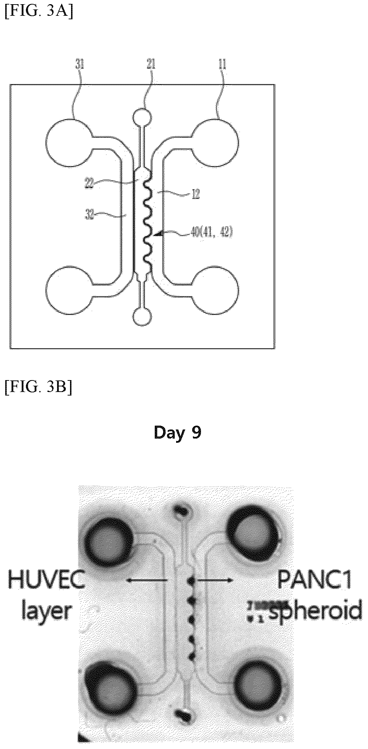

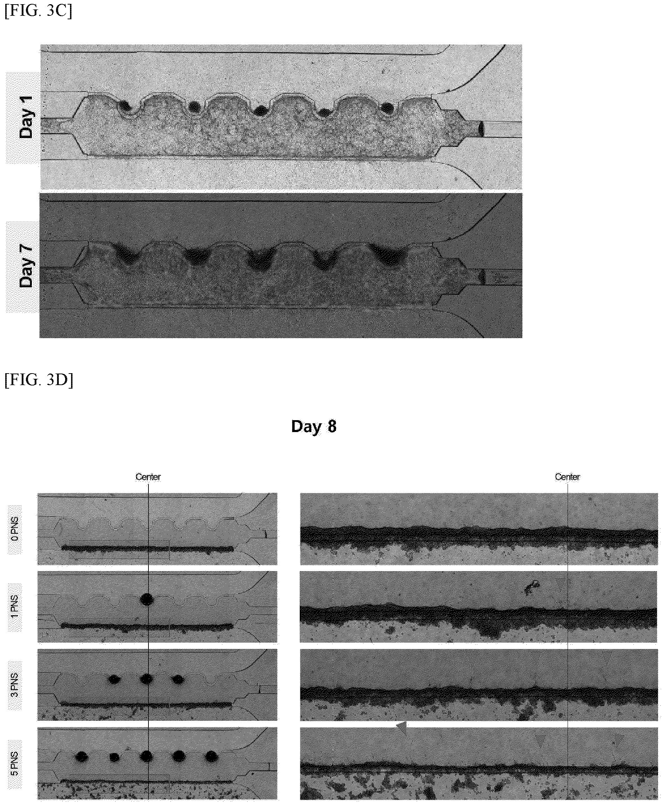

FIG. 3A schematically shows a microfluidic chip including an ECM channel 22 with concave patterns at one side according to one embodiment of the present invention, FIGS. 3B and 3C show pancreatic cancer cell spheroids, observed after co-culturing pancreatic cells as cells and vascular endothelial cells as second cells on the microfluidic chip for 7 to 9 days, and FIG. 3D shows the capturing locations and group numbers of peripheral nerve cell spheroids, observed after co-culturing peripheral nerve cells as cells and pancreatic cancer cells as second cells on the microfluidic chip for 8 days.

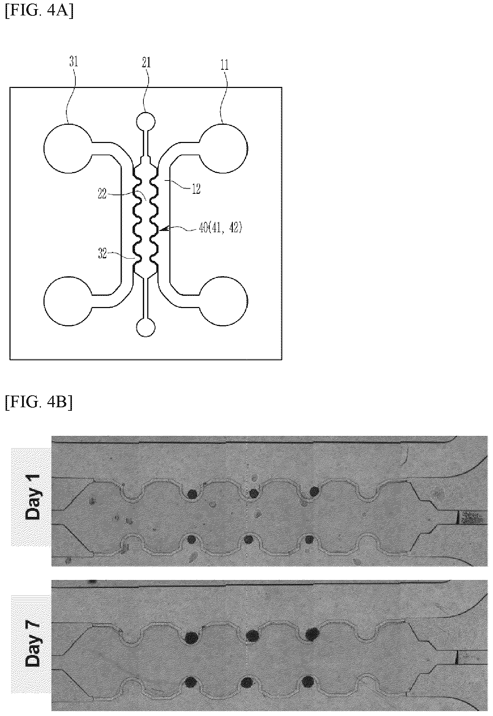

FIG. 4A schematically shows a microfluidic chip including an ECM channel 22 with concave patterns at both sides according to a second embodiment of the present invention, and FIG. 4B shows lung cancer cell organoids, observed after culturing lung cancer cells as cells and second cells on the microfluidic chip for 7 days.

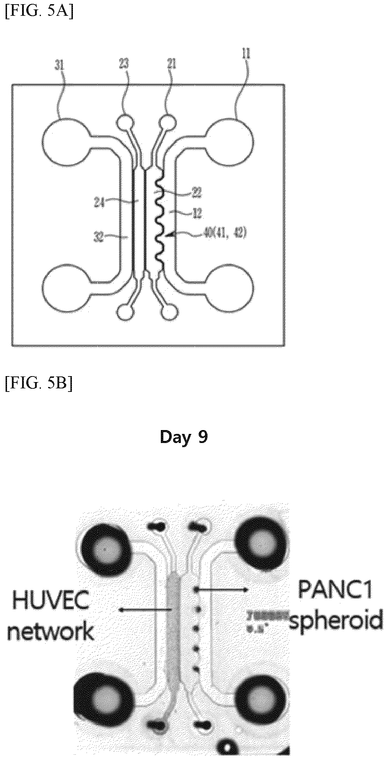

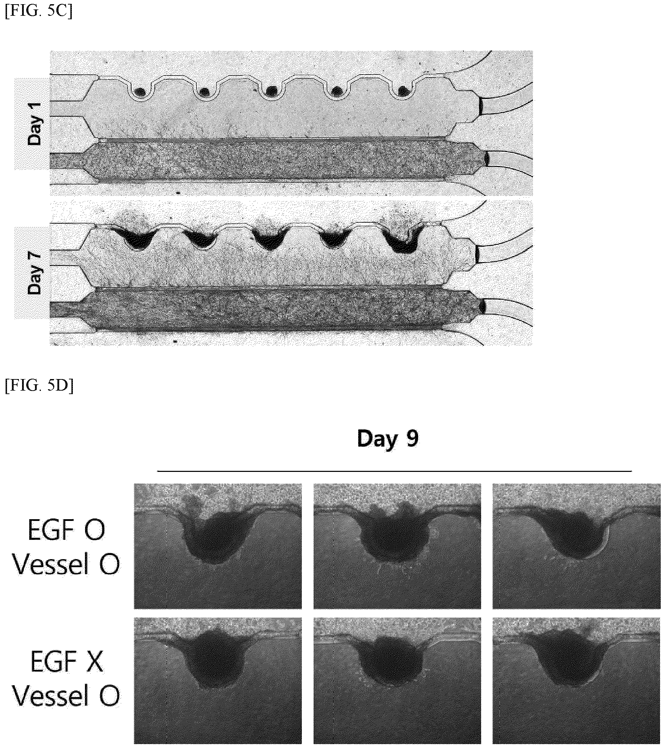

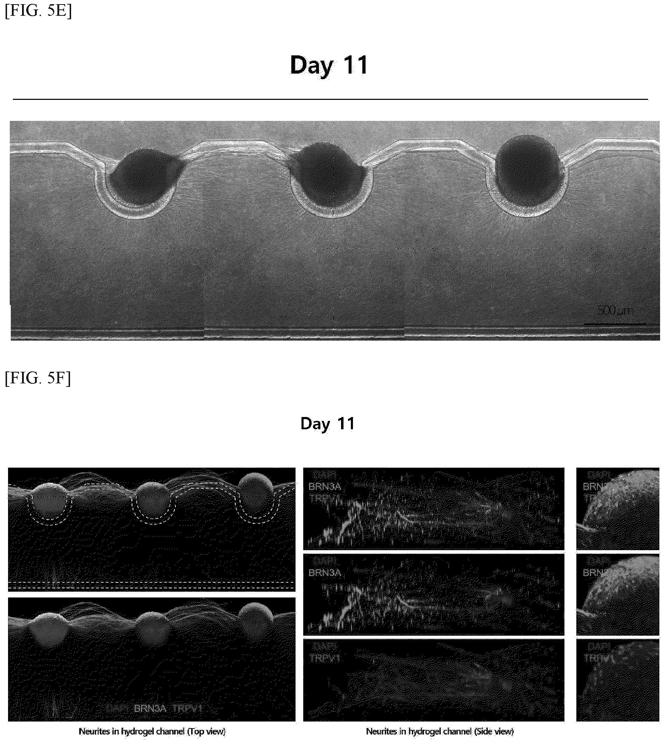

FIG. 5A schematically shows a microfluidic chip including an ECM channel 22 and a second ECM channel 24 according to a third embodiment of the present invention, FIGS. 5B to 5D show pancreatic cancer cell spheroids, observed after co-culturing pancreatic cancer cells as cells and vascular endothelial cells as second cells on the microfluidic chip for 7 to 9 days, and FIGS. 5E to 5F show peripheral nerve cell spheroids, observed after co-culturing peripheral nerve cells as cells and vascular endothelial cells as second cells on the microfluidic chip for 11 days.

### DETAILED DESCRIPTION OF EXEMPLARY EMBODIMENTS

When culturing spheroids and organoids in the conventional microfluidic chip, there was a limitation in controlling the number and locations thereof. To overcome this, a microfluidic chip using a micro barrier with concave patterns was designed and the height of the micro barrier and the diameter of the concave pattern were optimized, resulting in completing the present invention.

Hereinafter, the present invention will be described in detail.

### Microfluidic chip and method of producing spheroid or organoid in vitro model using the same

The present invention provides a spheroid or organoid microfluidic chip, which includes cell inlets into which a fluid containing cells is injected, and a cell channel connected thereto; and extra cellular matrix (ECM) inlets into which a fluid containing an ECM component is injected, and an ECM channel connected thereto. The cell channel is located adjacent to a part of one side of the ECM channel, and a micro barrier with a lower height than these channels is located between them and formed by arranging a plurality of concave patterns, curved in the direction of the ECM channel, at regular intervals.

The present invention also provides a method of producing a spheroid or organoid *in vitro* model, which includes (a) injecting a fluid containing cells into a cell inlet of a microfluidic chip; and (b) forming or culturing a spheroid or organoid with the injected cells at the locations of concave patterns that are arranged in the cell channel at regular intervals.

Hereinafter, embodiments of the present invention will be described in detail with reference to the accompanying drawings so that those of ordinary skill in the art can easily carry out the present invention. The present invention may be implemented in a variety of different forms, and is not limited to the embodiments described herein.

To clearly explain the present invention, parts that are not related to the explanation are omitted, and like numerals denote like parts throughout the specification.

In the following, when any component is said to be formed on the "upper part (or lower part)" of or "inside (or outside)" a substrate, it merely means that an arbitrary component is formed in contact with the upper part (or lower part) of or inside (or outside) the substrate, and does not mean that no other component is included between the substrate and the component formed on the upper part (or lower part) of the substrate or inside (or outside) the substrate.

Referring to FIGS. 1 and 3A, a microfluidic chip according to one embodiment of the present invention is configured to include cell inlets 11 into which a fluid containing cells is injected and a cell channel 12 connected thereto; and ECM inlets 21 into which a fluid containing an extra cellular matrix (ECM) component is injected and an ECM channel 22 connected thereto.

In addition, the cell channel 12 is located adjacent to a part of one side of the ECM channel 22, and a micro barrier 40 with a lower height than these channels is located between them and formed by arranging a plurality of concave patterns 41, curved in the direction of the ECM channel 22, at regular intervals.

Specifically, between the cell channel 12 and the ECM channel 22, where the micro barrier 40 is not placed, the cells and the ECM component are in direct contact and interact with each other. Accordingly, the height (h) of the micro barrier 40 may be 1/5 to 1/2, and preferably 1/5 to 2/5 of the height (H) of the channels, but the present invention is not limited thereto. The micro barrier 40 plays a supporting role to prevent the fluid containing the ECM component from leaking out of the ECM channel before being gelated after the injection of the fluid. When the height (h) of the micro barrier 40 is too low relative to the height (H) of the channels, there is a problem that the fluid containing the ECM component leaks out of the ECM channel before it is gelated after the injection of the fluid, and when the height (h) of the micro barrier 40 is too high relative to the height (H) of the channels, there is a problem that interaction is difficult due to a very small contact area between the channels.

In addition, in the micro barrier 40, the diameter (D) of the concave pattern 41 may be 1/5 to 1/3, and preferably 1/5 to 3/10 of the maximum width (W) of the ECM channel 22, but the present invention is not limited thereto. The diameter (D) of the concave pattern 41 may be a diameter when the concave pattern 41 is a semicircle, or may be the average of the major axis and the minor axis when the concave pattern 41 is a semi-ellipse. In addition, the maximum width (W) of the ECM channel 22 may be the longest width between the other side of the ECM channel 22 and the micro barrier 40 (that is, the width between the other side of the ECM channel 22 and the micro barrier 40 where a concave pattern 41 or a chamfer 42 is not formed). Here, in the micro barrier 40, when the diameter (D) of the concave pattern 41 is too large, the injected fluid may leak out of the ECM channel due to excessive pressure, or may not facilitate gel filling of the fluid containing the ECM component. On the other hand, in the micro barrier 40, when the diameter (D) of the concave pattern 41 is too small, it is not effective in capturing spheroids or organoids.

In the micro barrier 40, the gap between concave patterns 41 may range from 100 µm to 5,000 µm, preferably 300 µm to 3,000 µm, and more preferably 600 µm to 3,000 µm, but the present invention is not limited thereto. The gap between concave patterns 41 is larger than the diameter (D) of the concave patterns 41 in the micro barrier 40, and may be controlled by considering the amount of spheroid or organoid analysis.

Here, in the micro barrier 40, the concave pattern 41 may be connected to a chamfer 42 at both ends. The chamfer 42 may be designed to more easily capture spheroids or organoids and may be inclined toward the concave pattern 41. The chamfer 42 may be set to have a slope to reduce the shear stress exerted by the fluid on a cell body, thereby minimizing the effect on spheroid or organoid capture. In addition, the chamfer 42 is effective in gel filling of the fluid containing the ECM component. Specifically, the chamfer 42 may be inclined to the maximum extent up to the radius (or depth) (D/2) of the concave pattern 41, and may be inclined at an angle of 10° or more, and preferably 10° to 40°, but the present invention is not limited thereto.

In the fluid containing the cells, the cells may be may be cells to be cultured as spheroids or organoids, or may already be spheroid or organoid-type cells. For example, the cells may be cancer cells or neuronal cells. In a specific embodiment of the present invention, the cells may be pancreatic cancer cells, lung cancer cells, or peripheral nerve cells. In addition, the fluid may be any of various types of media for culturing cells, and in a specific embodiment of the present invention, a DMEM and EGM-2 mixed medium, a PNS medium, or an LTM medium was used.

In addition, the fluid containing the ECM component may be a fluid containing at least one selected from the group consisting of alginate, collagen, peptide, fibrin, hyaluronic acid, agarose, polyhydroxyethylmethacrylate (PHEMA), polyvinyl alcohol (PVA), poly(ethylene glycol) (PEG), polyethylene oxide) (PEO), polyethylene (glycol) diacrylate (PEGDA), gelatin, Matrigel, poly(L-lactic acid) (PLLA), carboxymethylcellulose, SAP, PHEMA-MMA, dextran, and chitosan, and preferably a fluid containing collagen, fibrin, or Matrigel, but the present invention is not limited thereto. Here, considering the viscosity of the fluid containing the ECM component, it may be a fluid containing collagen at 6 mg/ml or less, a fluid containing fibrin at 10 mg/ml or less, or a fluid containing Matrigel at 7 mg/ml or less.

Additionally, the microfluidic chip may further include a second cell channel 32 located adjacent to a part of the other side of the ECM channel 22, and the second cell channel 32 may be connected to a second cell inlet 31 into which a fluid containing second cells is injected. In addition, a second micro barrier (not shown in the drawing) with a lower height than the second cell channel 32 and the ECM channel 22 may be located between them. Therefore, between the second cell channel 32 where the second micro barrier (not shown in the drawing) is not located and the ECM channel 22, the second cells and the ECM component are in direct contact and interact with each other. Likewise, the height (h) of the second micro barrier (not shown in the drawing) may be 1/5 to 1/2, and preferably 1/5 to 2/5 of the height (H) of the channels, but the present invention is not limited thereto.

Selectively, referring to FIG. 4A, the second micro barrier (not shown in the drawing) may be formed by arranging a plurality of second concave patterns, curved in the direction of the ECM channel, at regular intervals. In the second micro barrier (not shown in the drawing), the diameter (D') of the concave pattern may be 1/5 to 1/3, and preferably 1/5 to 3/10 of the maximum width (W) of the ECM channel 22, but the present invention is not limited thereto. The diameter (D') of the second concave pattern may be the diameter when the second concave pattern is a semicircle, and may be the average of the major axis and the minor axis when the second concave pattern is a semi-ellipse. In addition, the maximum width (W) of the ECM channel 22 may be the largest width (i.e., the width of the distance between one side of the ECM channel 22 and the second micro barrier (not shown in the drawing) where the second concave pattern or chamfer is not formed) of the distance between one side of the ECM channel 22 and the second micro barrier (not shown in the drawing). Here, in the second micro barrier (not shown in the drawing), when the diameter (D') of the second concave pattern is too large, the injected fluid may leak out of the ECM channel due to excessive pressure, or may not facilitate gel filling of the fluid containing the ECM component. Meanwhile, in the second micro barrier (not shown in the drawing), when the diameter (D') of the second concave pattern is too small, it is not effective in capturing spheroids or organoids.

In the second micro barrier (not shown in the drawing), the gap between second concave patterns may range from 100 µm to 5,000 µm, preferably 300 µm to 3,000 µm, and more preferably 600 µm to 3,000 µm, but the present invention is not limited thereto. The gap between the second concave patterns is larger than the diameter (D') of the second concave pattern in the second micro barrier (not shown in the drawing), and may be controlled by considering the amount of spheroid or organoid analysis.

Here, in the second micro barrier (not shown in the drawing), both ends of the second concave pattern may be connected to a second chamfer. The second chamfer may be designed to more easily capture spheroids or organoids and inclined toward the second concave pattern. The second chamfer may be set to have a slope to reduce the shear stress exerted by the fluid on a cell body, thereby minimizing the effect on spheroid or organoid capture. In addition, the second chamfer 42 is effective in gel filling of the fluid containing the ECM component. Specifically, the second chamfer may be inclined to the maximum extent up to the radius (or depth) (D'/2) of the second concave pattern, and may be inclined at an angle of 10° or more, and preferably 10° to 40°, but the present invention is not limited thereto.

In the fluid containing the second cells, the second cells may be cells that can be co-cultured with the above cells and may be the same or different types of cells. For example, the second cells may be one or more types of co-culture cells selected from the group consisting of cancer cells, neuronal cells, and vascular endothelial cells. When the second cells are vascular endothelial cells, a vascular layer may be formed along the other side of the ECM channel 22 in the second cell channel 32. In a specific embodiment of the present invention, vascular endothelial cells, pancreatic cancer cells, or lung cancer cells were used. In addition, the fluid may be any of various types of media for culturing cells, and in a specific embodiment, a DMEM and EGM-2 mixed medium, a PNS medium, or an LTM medium was used.

Referring to FIG. 5A, a microfluidic chip according to another embodiment of the present invention is configured to include cell inlets 11 into which a fluid containing cells is injected and a cell channel 12 connected thereto; and ECM inlets 21 into which a fluid containing an ECM component is injected and an ECM channel 22 connected thereto.

In addition, the cell channel 12 is located adjacent to a part of one side of the ECM channel 22, and a micro barrier 40 with a lower height than these channels is located between them and formed by arranging a plurality of concave patterns 41, curved in the direction of the ECM channel 22, at regular intervals.

Since the cell inlet 11, the cell channel 12, the ECM inlet 21, the ECM channel 22, and the micro barrier 40 have been described, duplicated explanations will be omitted.

Additionally, a microfluidic chip according to one embodiment of the present invention may further include a fluid containing a second ECM component, and optionally, second ECM inlets 23 into which a fluid containing second cells is injected and a second ECM channel 24 connected thereto. A part of one side of the second ECM channel 24 may be located adj acent to a part of the other side of the ECM channel 22, and a second micro barrier (not shown in the drawing) with a lower height than the above channels may be placed between them.

Alternatively, a microfluidic chip according to one embodiment of the present invention may further include a second cell channel 32 located adjacent to a part of the other side of the second ECM channel 24, and the second cell channel 32 may be optionally connected to second cell inlets 31 into which a fluid containing second cells is injected. In addition, a third micro barrier (not shown in the drawing) with a lower height than the second cell channel 32 and the second ECM channel 24 may be placed between them.

Likewise, the height (h' or h") of the second or third micro barrier (not shown in the drawing) may be 1/5 to 1/2, and preferably 1/5 to 2/5 of the height (H) of the channels, but the present invention is not limited thereto.

The fluid containing a second ECM component may be the same as or different from the fluid containing the first ECM component and may be a fluid containing at least one selected from the group consisting of alginate, collagen, peptide, fibrin, hyaluronic acid, agarose, polyhydroxyethylmethacrylate (PHEMA), polyvinyl alcohol (PVA), poly(ethylene glycol) (PEG), polyethylene oxide) (PEO), polyethylene (glycol) diacrylate (PEGDA), gelatin, Matrigel, poly(L-lactic acid) (PLLA), carboxymethylcellulose, SAP, PHEMA-MMA, dextran, and chitosan, and preferably a fluid containing collagen, fibrin, or Matrigel, but the present invention is not limited thereto. Here, considering the viscosity of the fluid containing the ECM component, it may be a fluid containing collagen at 6 mg/ml or less, a fluid containing fibrin at 10 mg/ml or less, or a fluid containing Matrigel at 7 mg/ml or less.

In addition, in the fluid containing the second cells, the second cells may be cells that can be co-cultured with the cells and the same or different types of cells. For example, the second cells may be one or more co-culture cells selected from the group consisting of cancer cells, neuronal cells, and vascular endothelial cells. When the second cells are A vascular endothelial cells, they may penetrate into the second ECM channel 24 to form a net structure, thereby forming a vascular network. In a specific embodiment of the present invention, vascular endothelial cells, pancreatic cancer cells, or lung cancer cells were used. In addition, the fluid may be any of various types of media for culturing cells, and in a specific embodiment of the present invention, a DMEM and EGM-2 mixed medium, a PNS medium, or an LTM medium was used.

As described above, in the microfluidic chip according to the present invention, a micro barrier with a lower height than the cell channel and the ECM channel is located between them and formed by arranging a plurality of concave patterns, curved in the direction of the ECM channel, at regular intervals. Accordingly, by capturing and culturing spheroids or organoids using the concave patterns, the interaction between the spheroids or organoids and the ECM component may be observed between the cell channel and the ECM channel, where a micro barrier was not placed. In addition, using the concave patterns, spheroids or organoids may be captured and cultured.

Accordingly, upon the formation or culturing of spheroids and organoids, it is easy to control the number and locations of the spheroids and organoids, which can improve the reliability of analysis data and increase the convenience of analysis since it is sufficient to analyze only the predetermined location. Particularly, when capturing spheroids and organoids, the capturing numbers and locations of spheroids and organoids may be specified from the center of the concave patterns by controlling gravity and fluid flow.

Therefore, the spheroids and organoids cultured according to the present invention may be applied as animal replacement models for screening various drugs.

Hereinafter, preferred examples will be presented to allow the present invention to be better understood. However, the following examples are merely provided in order for the present invention to be more easily understood, and the content of the present invention is not limited by the following examples.

### [Examples]

### Examples 1: Optimal design of microfluidic chip having ECM channel 22

A spheroid/organoid microfluidic chip, which includes cell inlets 11 into which a fluid containing cells is injected and a cell channel 12 connected thereto; ECM inlets 21 into which a fluid containing an ECM component is injected and an ECM channel 22 connected thereto; and second cell inlets 31 into which a fluid containing second cells is injected and a second cell channel 32 connected thereto, was designed.

Here, the cell channel 12 is located adjacent to one side of the ECM channel 22, and a micro barrier 40 (h: 200 µm) with a lower height than the channels (H: 500 µm) is located between them. The micro barrier 40 is formed by arranging a plurality of concave patterns 41, curved in the direction of the ECM channel, at regular intervals (in terms of the diameter (D) of the concave pattern 41, at an interval of 1,600 µm based on the center point), and particularly, both ends of the concave pattern 41 are connected to different chamfers 42 formed at an angle of 30°. Meanwhile, between the cell channel 12 and the ECM channel 22 where the micro barrier is not placed, the cells and the ECM component can be in direct contact and interact with each other.

Meanwhile, the second cell channel 32 is located adjacent to a part of the other side of the ECM channel 22, and a second micro barrier (h': 200 µm; not shown in the drawing) with a lower height than the channels (H': 500 µm) is located between them. Meanwhile, between the second cell channel 32 and the ECM channel 22, where the second micro barrier is not placed, the second cells and the ECM component can be in direct contact and interact with each other.

If necessary, the second micro barrier is formed by arranging a plurality of second concave patterns, curved in the direction of the ECM channel, at regular intervals (in terms of the diameter (D) of the second concave pattern, at an interval of 1,600 µm based on the center point), and particularly, both ends of the second concave pattern are connected to different chamfers formed at an angle of 30°.

In addition, design changes were made to optimize the diameter of the concave pattern 41. As shown in Table 1 below, while designing a spheroid/organoid microfluidic chip by variously controlling the diameter (D) of the concave pattern 41 and the maximum width (W) of the ECM channel 22, it was observed whether a fluid containing the ECM component injected into the ECM inlet 21 gelated without leaking out of the ECM channel.

As a result, as confirmed in Table 1 and FIG. 2 below, when the diameter (D) of the concave pattern 41 was 1/5 to 1/3 of the maximum width (W) of the ECM channel 22 like in the first, second and fourth designs, it was confirmed that a fluid containing collagen type 1 (2 mg/mL) remained gelated without leaking out of the ECM channel. Particularly, like the first and second designs, the diameter (D) of the concave pattern 41 of 1/5 to 3/10 of the maximum width (W) of the ECM channel 22 was more desirable for maintaining gelation. On the other hand, like the third design, when the diameter (D) of the concave pattern 41 was 1/2 or more of the maximum width (W) of the ECM channel 22, it was confirmed that there was a problem where the injected fluid leaked out of the ECM channel.

**[Table 1]**

| | **First design** | **Second design** | **Third design** | **Fourth design** |
|---|---|---|---|---|
| Diameter (D) of concave pattern 41 (µm) | 300 | 300 | 500 | 500 |
| Maximum width (W) of ECM channel 22 (µm) | 1,000 | 1,500 | 1,000 | 1,500 |
| Whether or not gelation was maintained | ⊚ | ⊚ | X | ○ |

### Example 2: Optimal design of microfluidic chip having ECM channel 22 and second ECM channel 24

A spheroid/organoid microfluidic chip, which included cell inlets 11 through which a cell-containing fluid is injected and a cell channel 12 connected thereto; an ECM inlet 21 through which an ECM-containing fluid is injected and an ECM channel 22 connected thereto; second ECM inlets 23 into which a fluid containing a second ECM component is injected and a second ECM channel 24 connected thereto; and second cell inlets 31 into which a fluid containing second cells and a second cell channel 32 connected thereto, was designed.

Here, the cell channel 12 was located adjacent to a part of one side of the ECM channel 22, a micro barrier 40 (h: 200 µm) with a lower height than the channels (H: 500 µm) was located between them, the micro barrier 40 was formed by arranging a plurality of concave patterns 41, curved in the direction of the ECM channel, at regular intervals, and particularly, both ends of the concave pattern 41 were connected to different chamfers 42. Meanwhile, the diameter (D) of the concave pattern 41 and the maximum width (W) of the ECM channel 22 were 300 µm and 1,000 µm, respectively.

Meanwhile, a part of one side of the second ECM channel 24 is located adjacent to a part of the other side of the ECM channel 22, and a second micro barrier (h': 200 µm; not shown in the drawing) with a lower height than the channels is located between them. In addition, the second cell channel 32 is located adjacent to a part of the other side of the second ECM channel 24, and a third micro barrier (h": 200 µm; not shown in the drawing) with a lower height than the channels (H': 500 µm) is located between them.

### Example 3: Culture using microfluidic chip including ECM channel 22 with concave pattern at one side

(1) Referring to Example 1, a spheroid/organoid microfluidic chip with the optimized diameter of the concave pattern 41 was designed. Here, a micro barrier 40 has arranged concave patterns, but a second micro barrier (not shown in the drawing) does not have a separate pattern (FIG. 3A).
(2) Afterward, a fluid containing collagen type 1 (2 mg/mL) was injected into the ECM inlet 21 and gelated. A fluid (a 1:1 mixed medium of RPMI and EGM-2) containing vascular endothelial cells (HUVECs; 2,000,000 cells/mL) was then injected into a second cell inlet 31 to form a vascular layer. Meanwhile, pancreatic cancer cell spheroids were captured at the concave patterns 41 by injecting a fluid (1:1 mixed medium of DMEM and EGM-2) containing a total of 5 groups of spheroids made from approximately 1,000 pancreatic cancer cells (PANC1) into a cell inlet 11 and co-cultured for 9 days, which is shown in FIG. 3B.
(3) Meanwhile, except that a fluid containing fibrin (10 mg/mL) was injected into the ECM inlet 21, and a fluid (1:1 mixed medium of RPMI and EGM-2) containing vascular endothelial cells (HUVECs; 4,000,000 cells/mL) was injected to the second cell inlet 31, pancreatic cancer cell spheroids were captured in the same manner as (2) and co-cultured for 7 days, which is shown in FIG. 3C.
(4) Meanwhile, except that a fluid (PNS medium) containing pancreatic cancer cells (2,000,000 cells/mL) was injected into a second cell inlet 31 and a fluid (PNS medium) containing 0, 1, or a total of 3 or 5 groups of spheroids made from approximately 1,000 peripheral nerve cells was injected into a cell inlet 11, peripheral nerve cell spheroids were captured in the same manner as (2) and co-cultured for 8 days, which is shown in FIG. 3D.

As shown in FIG. 3D, it is confirmed that depending on the number of groups of the injected peripheral nerve cell spheroids, locations can be specified from the center of the concave patterns where peripheral nerve cell spheroids are captured, and the number of groups of peripheral nerve cell spheroids captured and cultured at the concave patterns can be controlled.

### Example 4: Culture using microfluidic chip including ECM channel 22 with concave patterns at both sides

(1) Referring to Example 1, a spheroid/organoid microfluidic chip in which the diameter of a bilateral concave pattern was optimized was designed. Here, in both a micro barrier 40 and a second micro barrier (not shown in the drawing), second concave patterns are arranged (FIG. 4A).
(2) Afterward, a fluid containing collagen type 1 (2 mg/mL) was injected into an ECM inlet 21 and then gelated. Subsequently, a fluid (LTM medium) containing a total of three groups of organoids made from approximately 1,000 patient-derived lung cancer cells was injected into cell inlets 11 and second cell inlets 31 to capture the cells at concave patterns 41 and second concave patterns and culture the cells for 7 days, which is shown in FIG. 4B.

### Example 5: Culture using microfluidic chip including ECM channel 22 and second ECM channel 24

(1) Referring to Example 2, a spheroid/organoid microfluidic chip with the optimized diameter of concave patterns 41 was designed (FIG. 5A).
(2) Afterward, a fluid containing collagen type 1 (2 mg/mL) was injected into an ECM inlet 21 and then gelated, and a fibrin (10 mg/mL)-containing fluid was injected into a second ECM inlet 23 and a fluid (1:1 mixed medium of RPMI and EGM-2; 4,000,000 cells/mL) containing vascular endothelial cells (HUVECs) was injected into a second cell inlet 31 and each was gelated, thereby forming a vascular network. Meanwhile, a fluid (1:1 mixed medium of DMEM and EGM-2) containing a total of 5 groups of spheroids made from approximately 1,000 pancreatic cancer cells (PANC1) and optionally EGF (50 ng/mL) was injected into a cell inlet 11 to capture pancreatic cancer cell spheroids at concave patterns 41 and co-culture them for 7 to 9 days, which is shown in FIGS. 5B to 5D.
   As shown in FIGS. 5B to 5D, it is confirmed that, upon co-culture for 7 to 9 days, the pancreatic cancer cell spheroids were captured in the concave patterns curved in the direction of the ECM channel and uniformly cultured. Particularly, when EGF was added, it can be observed that the migration of pancreatic cancer cells was induced, leading to active invasion.
(3) Meanwhile, except that a fluid (1:1 mixed medium of RPMI and EGM-2) containing vascular endothelial cells (HUVEC; 4,000,000 cells/mL) was injected into a second cell inlet 31, and a fluid (PNS medium) containing a total of three groups of spheroids made from approximately 1,000 peripheral nerve cells was injected into the cell inlet 11, peripheral neuronal cell spheroids were captured in the same manner as (2) and cultured for 11 days, which is shown in FIGS. 5E and 5F.

As shown in FIGS. 5E and 5F, as a result of co-culture for 11 days, it is confirmed that peripheral neuronal cell spheroids are captured in concave patterns curved in the direction of the ECM channel, and peripheral nerve cells are uniformly cultured while their axons extend toward an ECM channel. Here, BRN3A (green signal) can be used to confirm sensory nerve cells, and TRPV1 (red signal) can be used to confirm peripheral nerve cells.

It should be understood by those of ordinary skill in the art that the above descriptions of the present invention are exemplary, and the embodiments disclosed herein can be easily modified into other specific forms without changing the technical spirit or essential features of the present invention. Therefore, it should be interpreted that the embodiments described above are exemplary in all aspects, and are not limitative.

## Claims

1. A spheroid or organoid microfluidic chip, comprising:
cell inlets (11) into which a fluid containing cells is injected, and a cell channel (12) connected thereto; and
extra cellular matrix (ECM) inlets (21) into which a fluid containing an ECM component is injected, and an ECM channel (22) connected thereto,
wherein the cell channel (12) is located adjacent to a part of one side of the ECM channels (22), and a micro barrier (40) with a lower height than the channels (12, 22) is located between them and formed by arranging a plurality of concave patterns (41), curved in the direction of the ECM channel (22), at regular intervals.

2. The microfluidic chip of claim 1, wherein, between the cell channel (12) and the ECM channel (22), where the micro barrier (40) is not placed, the cells and the ECM component interact with each other.

3. The microfluidic chip of claim 1 or 2, wherein the height of the micro barrier (40) is 1/5 to 1/2 of the height of the channels (12, 22).

4. The microfluidic chip of any preceding claim, wherein, in the micro barrier (40), both ends of a concave pattern (41) are connected to different chamfers (42).

5. The microfluidic chip of any preceding claim, wherein, in the micro barrier (40), the diameter of a concave pattern (41) is 1/5 to 1/3 of the maximum width of the ECM channel (22).

6. The microfluidic chip of any preceding claim, wherein, in the micro barrier (40), the gap between concave patterns (41) ranges from 100 µm to 5,000 µm.

7. The microfluidic chip of any preceding claim, wherein the cells are cancer cells or neuronal cells.

8. The microfluidic chip of any preceding claim, further comprising a second cell channel (32) located adjacent to a part of the other side of the ECM channel (22).

9. The microfluidic chip of claim 8, wherein, between the second cell channel (32) and the ECM channel (22), a second micro barrier with a lower height than these channels (32, 22) is located and formed by arranging a plurality of concave patterns (41), curved in the direction of the ECM channel (22), at regular intervals.

10. The microfluidic chip of claim 8 or 9, wherein the second cells are the same or different types of the above cells, and one or more types of co-culturing cells selected from the group consisting of cancer cells, neuronal cells, and vascular endothelial cells.

11. A method of producing a spheroid or organoid *in vitro* model, comprising:
(a) injecting a fluid containing cells into a cell inlet (111) of the microfluidic chip of any preceding claim; and
(b) forming or culturing a spheroid or organoid with the injected cells at the locations of concave patterns (41) that are arranged in the cell channel (12) at regular intervals.
